# EUROPEAN PATENT APPLICATION

(11) **EP 4 223 212 A1**
(43) Date of publication of application: **09.08.2023**
(21) Application number: 22154914.0
(22) Date of filing: 03.02.2022
(51) Int. Cl.: A61B 5/00, A61N 1/36

(54) **APPARATUS, METHODS AND COMPUTER PROGRAMS FOR GENERATING PAIN PROFILES**

(71) Applicant: Nokia Technologies Oy, 02610 Espoo (FI)
(72) Inventor: ZHENG, Mingde, Basking Ridge, NJ, 08807 (US); SAMANTA, Bibek, Piscataway, NJ, 08854 (US)
(74) Representative: Nokia EPO representatives

(57) **Abstract**

Examples of the disclosure relate to systems and apparatus that enable a pain profile to be generated. The apparatus comprises means for enabling stimulation of a biological sample using a plurality of electrical stimulating signals. The plurality of electrical stimulating signals comprise different values for one or more parameters and respective electrical stimulating signals are configured to cause a respective change in the biological sample wherein the respective change is optically detectable. The apparatus is also configured to enable measuring of the optically detectable changes caused by the electrical stimulating signals. The optically detectable changes can be measured by using one or more optical devices to detect the optically detectable changes. The measurements obtained by the one or more optical devices and the corresponding values for the one or more parameters of the electrical stimulating signals can be used to generate a pain profile for the biological sample. The pain profile is based on the optically detectable changes caused by the stimulation.

## Description

### TECHNOLOGICAL FIELD

Examples of the disclosure relate to apparatus, methods and computer programs for generating profiles. Some relate to apparatus, methods and computer programs for generating profiles that assign a quantitate value to pain experienced by a subject.

### BACKGROUND

How a subject such as a person or animal responds to pain varies between different subjects. This makes it difficult to make comparisons of pain levels between different subjects or to make an assessment of pain levels for a subject that cannot communicate their pain levels.

### BRIEF SUMMARY

According to various, but not necessarily all, examples of the disclosure there may be provided an apparatus comprising means for:
enabling stimulation of a biological sample using a plurality of electrical stimulating signals comprising different values for one or more parameters wherein respective electrical stimulating signals are configured to cause a respective change in the biological sample wherein the respective change is optically detectable;
enabling measuring of the optically detectable changes caused by the electrical stimulating signals by using one or more optical devices to detect the optically detectable changes; and
using the measurements obtained by the one or more optical devices and the corresponding values for the one or more parameters of the electrical stimulating signals to generate a pain profile for the biological sample based on the optically detectable changes caused by the stimulation.

The one or more optical devices used to detect the optically detectable changes may comprise one or more optical coherence tomography devices.

The measurements obtained by the one or more optical devices may be used to generate the pain profile for a region of the pain profile below a threshold level of the electrical stimulating signal and extrapolation of data may be used to generate the pain profile for a region above the threshold level of the electrical stimulating signal.

The one or more parameters of the electrical stimulating signals may comprise power.

The generated pain profile may be specific to the biological sample.

The stimulation of the biological sample may be spatially modulated so as to target one or more regions of the biological sample.

The the respective optically detectable changes caused by the electrical stimulating signals may comprise one or more cellular or biomolecular level events.

The cellular or biomolecular level events may comprise one or more of; cellular depolarization, transmembrane voltage changes, changes in nervous tissue, electrolytic chemical imbalance.

The optically detectable changes may comprise a change in one or more optical properties of the biological sample.

The optical properties may comprise one or more of: refractive index, birefringence, fluorescence.

The one or more optical devices may comprise a polarization-sensitive device.

The values for the one or more parameters of the electrical stimulating signals may be applied at a level that is controlled by the subject.

The measurements obtained by the one or more optical devices and the corresponding values for the one or more parameters of the electrical stimulating signals may be used in combination with one or more qualitative pain indicators to generate the pain profile for the biological sample

The means may be for controlling the electrical stimulating signals to vary one or more of frequency, duration, cycle, pattern, shape of the electrical stimulating signals.

The optical device may comprise a plurality of optical sensors configured to be positioned around the biological sample.

The apparatus may comprise means for obtaining observation measurements from an optical device and using the obtained observation measurements and the generated pain profile to determine a pain level of the biological sample.

According to various, but not necessarily all, examples of the disclosure there may be provided an electronic device comprising an apparatus as described herein

According to various, but not necessarily all, examples of the disclosure there may be provided a method comprising:
enabling stimulation of a biological sample using a plurality of electrical stimulating signals comprising different values for one or more parameters wherein respective electrical stimulating signals are configured to cause a respective change in the biological sample wherein the respective change is optically detectable;
enabling measuring of the optically detectable changes caused by the stimulating signals by using one or more optical devices to detect the optically detectable changes; and
using the measurements obtained by the one or more optical devices and the corresponding values for the one or more parameters of the electrical stimulating signals to generate a pain profile for the biological sample based on the optically detectable changes caused by the stimulation.

According to various, but not necessarily all, examples of the disclosure there may be provided a computer program comprising computer program instructions that, when executed by processing circuitry, cause:
enabling stimulation of a biological sample using a plurality of electrical stimulating signals comprising different values for one or more parameters wherein respective electrical stimulating signals are configured to cause a respective change in the biological sample wherein the respective change is optically detectable;
enabling measuring of the optically detectable changes caused by the stimulating signals by using one or more optical devices to detect the optically detectable changes; and
using the measurements obtained by the one or more optical devices and the corresponding values for the one or more parameters of the electrical stimulating signals to generate a pain profile for the biological sample based on the optically detectable changes caused by the stimulation.

### BRIEF DESCRIPTION

Some examples will now be described with reference to the accompanying drawings in which:
FIGS. 1A and 1B schematically shows an example system and biological sample;
FIG. 2 shows an example method that can be implemented using the example system;
FIG. 3 shows another example system;
FIG. 4 show a biological sample being scanned;
FIG. 5 shows an example pain profile; and
FIG. 6 shows an example apparatus.

### DETAILED DESCRIPTION

It can be useful to determine a level of pain experienced by a subject. For instance, a medical practitioner or caregiver might want to assess the pain experienced by a subject, such as a person or animal, in their care. In some cases the medical practitioner or caregiver might be able to communicate with the subject to obtain an indication of the pain being experienced. However, this is unreliable as different subjects will respond differently to the similar pain levels. Examples of the disclosure therefore provide a system that enables a pain profile to be generated. The pain profile can be based on a detectable biophysical response of the subject to external stimuli. Once the pain profile has been generated it can be used as a reference so that physical characteristics of the subject can be monitored and then compared to the pain profile to give a more reliable indication of the pain being experienced by the subject.

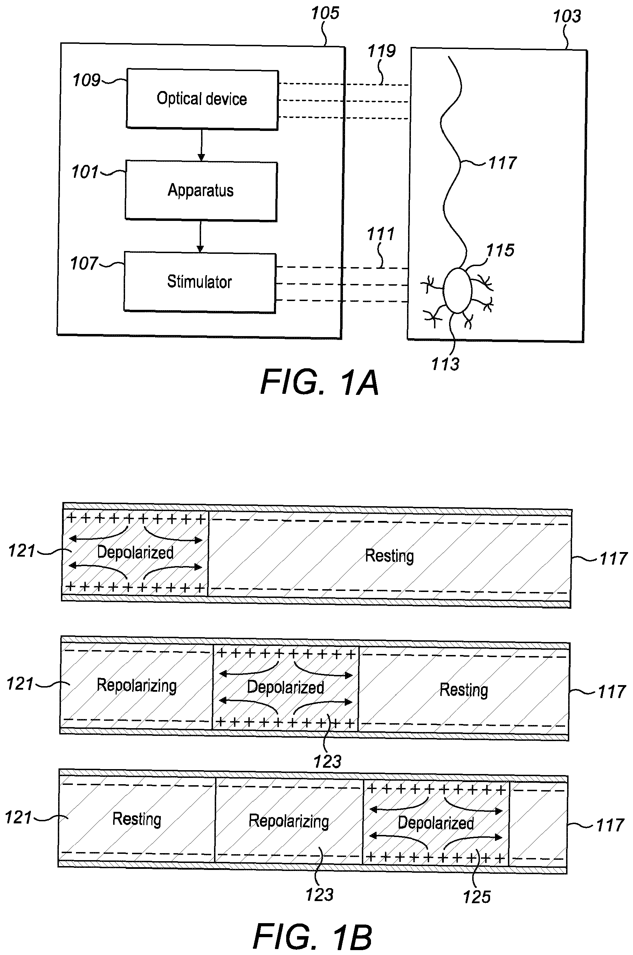

Fig. 1A schematically shows an example system 105 that can be used to implement examples of the disclosure. The example system 105 comprises an apparatus 101, at least one stimulator 107 and at least one optical device 109. The example device can also comprise additional components that are not shown in Fig. 1A.

The apparatus 101 can be configured to control the system 105. In the example of Fig. 1A the apparatus 101 is shown inside the system 105. In other examples the apparatus 101 could be provided external to the system 105 and could be configured to provide control signals to the system 105 remotely.

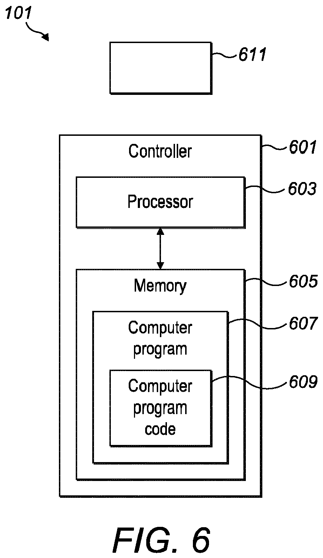

The apparatus 101 can be a controller apparatus 101 or any other suitable type of apparatus 101. An example of a controller apparatus 101 is shown in Fig. 6. The apparatus 101 can be implemented as controller circuitry. In some examples the apparatus 101 can be implemented in hardware alone, have certain aspects in software including firmware alone or can be a combination of hardware and software (including firmware). The apparatus 101 can be configured to control the stimulator 107 and/or the optical device 109.

The stimulator 107 can comprise any means that can be configured to provide a plurality of electrical stimulating signals 111 for stimulating a biological sample 103. The electrical stimulating signals 111 can comprise any signal that can stimulate the biological sample 103 so as to cause an optically detectable change within the biological sample 103. The electrical stimulating signals 111 can be configured to cause a change at a cellular or molecular level of the biological sample 103. The electrical stimulating signals 111 can provide an external stimulus to the biological sample 103 that causes a pain response within the biological sample 103.

The electrical stimulating signals 111 can be provided at a plurality of different powers. The plurality of different powers can cover a range that comprises electrical stimulating signals 111 that are not perceptible to a subject, electrical stimulating signals 111 that are barely perceptible to a subject, electrical stimulating signals 111 that cause a small level of discomfort to a subject and/or any other suitable electrical signal levels 111.

The apparatus 101 can be configured to control the stimulator 107 to provide the electrical stimulating signals 111 in programmed waveforms. The timing, power, frequency and any other suitable property of the electrical stimulating signals 111 can be controlled through the apparatus 101.

The stimulator 107 can comprise means that enables the electrical stimulating signals 111 to be provided to the biological sample 103. In some examples the means can comprise one or more electrodes that can be applied to the biological sample 103. In some examples the electrodes can be galvanically connected to the biological sample 103 to provide a direct current path for the electrical signals from the electrodes into the biological sample 103.

In some examples the stimulator 107 can comprise a plurality of electrodes and/or sets of electrodes that can be positioned on different parts of the biological sample 103. This can enable different parts of the biological sample 103 to be targeted using the electrical stimulating signals 111.

The biological sample 103 can comprise a subject or part or a subject. The subject can be any subject that can feel pain. For example, the subject could be a person or an animal. Only part of the biological sample 103 is shown in Fig. 1A.

The biological sample 103 comprises a plurality of sensory receptors 113. The sensory receptors 113 can comprise nerve cells or any other suitable type of receptors that can provide an optically detectable response to the electrical stimulating signals 111. In the example of Fig. 1A only one sensory receptor 113 is shown however it is to be appreciated that the biological sample 103 could comprise any number of the sensory receptors 113.

The sensory receptors 113 can be located directly underneath skin of the biological sample 103 and/or deeper within the biological sample 103. The sensory receptors 113 can convert internal stimuli such as inflammation, external stimuli such as pressure or temperature, and/or the electrical stimulating signals 111 into pain signals that can be detected neurologically by the subject. The plurality of sensory receptors 113 can enable the subject to feel pain.

The sensory receptors 113 can comprise a soma 115 and an axon 117. The sensory receptors 113 can respond to the electrical stimulating signals 111 or other external stimuli and propagate the pain signals as an impulse from the soma 115 along the axon 117 and towards the central nervous system or brain of the subject. The impulse comprises a sequence of electrochemical processes caused by the depolarization of ions within cells inside and outside of the axons 117.

Fig. 1B shows how the pain signals can propagate through the axon 117 of the sensory receptors 113. Fig. 1B schematically shows the same section of an axon 117 at three sequential points in time.

In response to an external stimulus such as the electrical stimulating signals 111 the end of the axon 117 closest to the soma 115 becomes depolarized. That creates a depolarized section in the first part 121 of the axon 117.

The depolarization moves along the axon 117 so that other sections of the axon 117 become depolarized. Once the second part 123 of the axon 117 adjacent to the first part 121 has become depolarized, the first part 121 of the axon 117 now repolarizes. The repolarization is caused by the positive sodium ions being inactivated and additional channels for the positive potassium ions being opened. Once the third part 125 of the axon 117 adjacent to the second part 123 has become depolarized, the second part 123 of the axon 117 now repolarizes and the first part 121 returns to a resting state. This sequence continues along the length of the axon 117.

The depolarization and repolarization of the sections of the axon 117 is a transient effect. It lasts for a short period of time as the pain signals travel along the axon 117.

The depolarization and repolarization of the sections of the axon 117 forms the impulse patterns that the brain can interpret as pain. These impulses produce optically detectable changes within the biological sample 103. The depolarization and repolarization of the sections of the axon 117 produce optically detectable changes in the region of the biological sample 103 around the sensory receptors 113 that have been stimulated by the external stimuli such as the electrical stimulating signals 111.

The optical device 109 of the system 105 can be configured to detect the optically detectable changes within the biological sample 103. In the example of Fig. 1A one optical device 109 is shown. In other examples the system 105 could comprise a plurality of optical devices 109.

The optical device 109 can be configured to detect light 119 reflected or scattered from the biological sample 103. In some examples the optical device 109 can comprise one or more emitters that are used to emit light and one or more light sensors that can detect light reflected or scattered from the biological sample 103. The emitters can be configured to enable light from the optical device 109 to be used to scan the biological sample 103. The emitters and sensors can be positioned to enable a particular section of the biological samples 103 to be monitored. Using emitters and sensors in different positions can enable different sections of the biological sample 103 to be monitored.

In some examples the optical device 109 can comprise one or more optical coherence tomography (OCT) devices or any other suitable type of device. The OCT device can use near infra-red light to scan the biological sample 103.

In some examples the optical device 109 can be a polarization sensitive device. This can enable the optical device 109 to detect changes in polarization of the light 119 from the biological sample 103.

The optical device 109 can be configured to detect optical changes that would not be visible to the naked eye. The optically detectable changes can comprise a change in one or more optical properties of the biological sample 103. The optical properties can comprise one or more of, refractive index, birefringence, fluorescence or any other suitable optical property. The optically detectable changes can comprise changes that are internal to the biological sample 103, for example they can comprise changes to one or more internal structures within the biological sample 103.

The optical device 109 can be configured to enable the optically detectable changes of the biological sample 103 to be measured. For example, the change in the refractive index, birefringence, fluorescence or any other suitable optical property could be measured from the light 119 reflected or scattered from the biological sample and detected by the optical device 109. These measurements can provide an indication of the response of the sensory receptors 113 to the electrical stimulating signals 111 at different prescribed levels. This can enable a pain profile to be generated for the biological sample 103 based on the optically detectable changes and the corresponding power of the electrical stimulating signals 111.

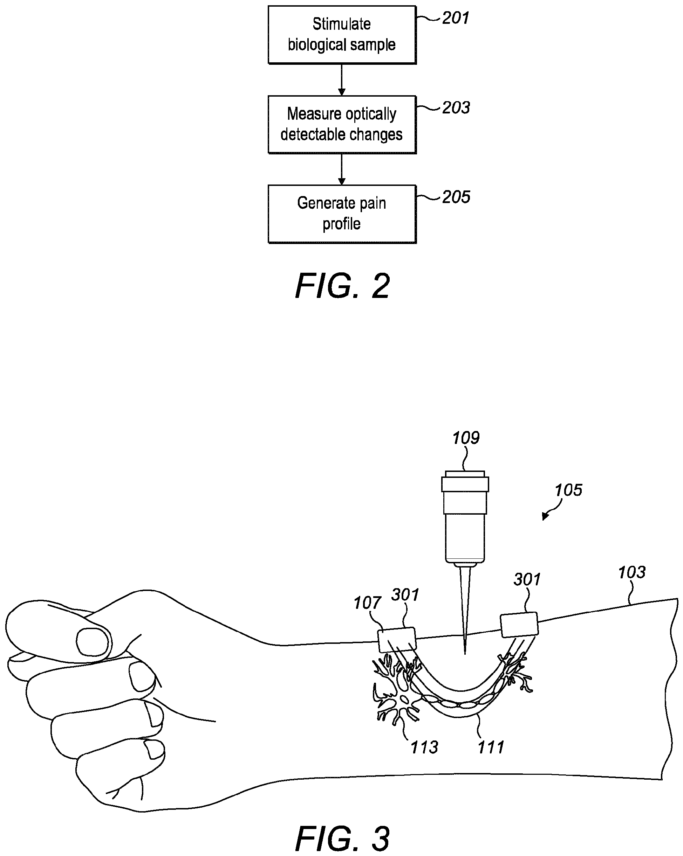

Fig. 2 shows an example method for generating a pain profile that can be implemented using the example apparatus 101 and system 105 as shown in Fig. 1A.

The method comprises, at block 201, enabling stimulation of the biological sample 103. The apparatus can enable stimulation of the biological sample 103 by controlling the stimulator 107 to provide the electrical stimulating signals 111 to the biological sample 103. A plurality of electrical stimulating signals 111 can be used to stimulate the biological sample 103. The apparatus 101 can be configured to control the stimulator 107 to provide the electrical stimulating signals 111 at different power levels.

The apparatus 101 can be configured to control any suitable parameters of the electrical stimulating signals 111. For example, the apparatus 101 can be configured to vary one or more of frequency, duration, cycle, pattern, shape, amplitude of the electrical stimulating signals 111 and/or any other suitable parameter. The apparatus 101 can be configured to provide different electrical stimulating signals 111 with different respective values for one or more of the parameters. This variation in the parameters can enable a multi phasic stimulation to be provided to the biological sample 103. The multi phasic stimulation can enable different types of stimulation to be provided to the biological sample 103, and/or can enable the stimulation to be provided to different parts of the biological sample 103. The multi phasic stimulation can enable different parts of the nervous tissue to be stimulated which can enable different responses of the biological sample 103 to be generated.

The electrical stimulating signals 111 cause optically detectable changes within the biological sample. The optically detectable changes can be caused by pain signals passing through the sensory receptors 113 within the biological sample 103.

The optically detectable changes caused by the electrical stimulating signals 111 can comprise one or more cellular or biomolecular level events. The cellular or biomolecular level events can comprise one or more of; cellular depolarization, transmembrane voltage changes, chemical changes in nervous tissue, electrolytic ion imbalance. For example, the optically detectable changes can comprise the depolarization and repolarization of the axon 117 of the sensory receptors 113 as a pain signal travels along the sensory receptors 113 or any other suitable change.

Different electrical stimulating signals 111 cause different changes within the biological sample 103. For instance, electrical stimulating signals 111 of different powers will cause different levels of optically detectable changes within the biological sample 103. In some examples, the more intense the electrical stimulating signals 111, the greater the optically detectable change in the biological sample 103 that is caused by the electrical stimulating signals 111.

At block 203 the method comprises enabling measuring of the optically detectable changes caused by the electrical stimulating signals 111. The apparatus 101 can enable the measuring of the optically detectable changes by controlling the optical device 109. For instance, in some examples the optical device could comprise an optical coherence tomography (OCT) device. In such examples the apparatus 101 can enable the measuring of the optically detectable changes by controlling the OCT device to scan the biological sample 103 and/or to scan a part of the biological sample 103.

The optically detectable changes can be measured by using the one or more optical devices 109 to detect the optically detectable changes. The measurement can assign one or more quantities to the detected optical changes.

The optically detectable changes that are caused within the biological sample 103 can be dependent upon the power, or other parameter of the electrical stimulating signals 111 that are used to stimulate the biological sample 103. For instance, a more powerful or intense electrical stimulating signal 111 can cause a higher pain response within the biological sample 103. This can cause a greater optically detectable change within the biological sample 103 which can be measured by the optical device 109.

At block 205 the method comprises using the measurements obtained by the one or more optical devices 109 and the corresponding values of the parameters such as the power of the electrical stimulating signals 111 to generate a relational reference. This relational reference maps or associates each electrical stimulation power, or value of other parameter, with a corresponding optical measurement, forming a pain profile for the biological sample 103. The pain profile is based, at least in part, on the optically detectable changes cause by the stimulation. The generated pain profile can correlate pain levels to the optically detectable changes within the biological sample 103.

To generate a pain profile a plurality of electrical stimulating signals 111 can be provided at a range of different powers. The plurality of different powers can cover a range that comprises electrical stimulating signals 111 that are not perceptible to a subject, electrical stimulating signals 111 that are barely perceptible to a subject, electrical stimulating signals 111 that cause a small level of discomfort to a subject and/or any other suitable signal levels. For instance, the electrical stimulating signals 111 can start at a very low power being transferred by the electrical signals and then can be increased to a higher level of power being transferred by the electrical signals. The different levels of power being transferred by the electrical signals can cause different intensities of pain for the subject.

The electrical stimulating signals 111 can be provided at a level that won't cause damage to the biological sample 103 or cause significant discomfort to the subject. In some examples the electrical stimulating signals 111 can have a stimulation current amplitude level well below 100 micro amperes.

The values for the one or more parametersof the electrical stimulating signals 111 can be applied at a level that can be controlled by the subject that the biological sample 103 is part of and/or by a medical practitioner or by any other suitable user. The values for the one or more parametersof the electrical stimulating signals 111 can be controlled, for example, so that they can be stopped before the power level and/or intensity of the pain becomes too uncomfortable for the subject.

The measurements of the optically detectable changes provide an indication of how the biological sample 103 responds to different electrically stimulated pain signals. The optically detectable changes that occur for different thresholds of power of the electrical stimulating signals 111 can be used to define different ranges within the pain profile. For instance, the optically detectable changes that occur for very low power signals can be classed in a first range, optically detectable changes that occur for low power signals can be classed in a second range and the optically detectable changes that occur for slightly higher power signals can be classed in a third range. Other ranges can be used in other examples.

In some examples feedback from the subject can also be used to help to generate the pain profile. For instance, as the electrical stimulating signals 111 are being applied the subject can be asked to indicate their level of discomfort. For example, the subject can indicate whether or not they feel no pain or a small amount of pain or a moderate amount of pain. The feedback from the subject can comprise one or more qualitative pain indicators. The qualitative pain indicators could comprise facial expressions, verbal indications or any other suitable indicators. The qualitative pain indicators could be used in combination with the measurements obtained by the one or more optical devices 109 to generate the pain profile.

The measurements obtained by the one or more optical devices 109 are used to generate the pain profile for a region of the pain profile below a threshold level of the electrical stimulating signal 111. Extrapolation of data can be used to generate the pain profile for a region above the threshold level of the electrical stimulating signal 111. This avoids the subject having to experience any discomfort when generating the pain profile. Any suitable process can be used to extrapolate the data. For instance, in some examples the expected optically detectable changes for higher levels of pain can be extrapolated using in-vitro measurements from a similar type of biological sample 103 and/or from literature references and/or any other suitable source and/or from a combination of these.

The generated pain profile can be specific to the biological sample 103 and/or the subject of which the biological sample 103 is a part of. For instance, by applying the electrical stimulating signals 111 to a subject and measuring the optically detectable changes, a pain profile unique to the biological sample 103 can be generated. In other examples the pain profile could be generated for a group or set of subjects.

Once the pain profile has been generated it can be used to provide an indication of pain levels being experienced by a subject. For instance, the subject could be observed by scanning the subject using the optical device 109. This can enable any optical changes within the subject to be detected. These optical changes can then be compared to the pain profile to identify the level of pain being experienced by the subject. In some examples different parts of the subject could be scanned using the optical device 109. This could enable a location of the pain to be identified as well as providing an indication of the level of pain.

Fig. 3 shows an example system 105 being used to generate a pain profile. The system 105 comprises a stimulator 107 and an optical device 109. The system 105 could also comprise an apparatus 101 which is not shown in Fig. 3. The apparatus 101 could be configured to control the various components of the system 105.

In the example of Fig. 3 the biological sample 103 that is being analysed by the system 105 is the forearm of a person. Other parts of a person and/or animal could be monitored and/or analysed in other examples of the disclosure.

In the example of Fig. 3 the stimulator 107 comprises a plurality of electrodes 301. The electrodes 301 are positioned on the forearm of the subject and enable electrical stimulating signals 111 to be provided to the biological sample 103. In this example the stimulator 107 comprises two electrodes 301 and the electrical stimulating signals 111 pass from a first electrode 301, through the biological sample 103 and to the second electrode 301. The position of electrodes 103 can therefore determine which parts of the biological sample 103 are stimulated by the electrical stimulating signals 111.

The electrodes 301 can comprise part of a wearable device.

The electrodes can comprise of 2-dimensional materials such as graphene or thin layers of metal or any other suitable conductive materials. The electrodes can be flexible so that they can fit to the skin of the subject.

In some examples, different parts of the biological sample 103 can be stimulated and analysed by positioning the electrodes 301 in different positions on the biological sample 103. For instance, the electrodes 301 can be positioned on the subject's forearm for a first set of measurements and then could be positioned on the subject's upper arm or the back of their hand or in any other suitable location for another set of measurements.

In some examples the stimulator 107 can comprise more than two electrodes 301 that can be positioned on the biological sample 103. This can enable different parts of the biological sample 103 to be stimulated by using different pairs of electrodes 301 to provide the electrical stimulating signals 111. The use of different electrodes 301 at different positions can allow for spatial modulation of the electrical stimulating signals 111.

The electrical stimulating signals 111 stimulate the biological sample to generate a pain response within the sensory receptor 113. One example sensory receptor 113 is shown in Fig. 3. It is to be appreciated that any number of sensory receptors 113 could be provided in the biological sample 103. Also, the sensory receptor 113 is not shown to scale in Fig. 3.

The electrical stimulating signals 111 produce optically detectable changes within the biological sample 103. These can be caused by the pain impulse travelling along the sensory receptor 113 or by any other factor.

The stimulator 107 can be controlled so that the electrical stimulating signals 111 penetrate into the biological sample 103. For example, the electrical stimulating signals 111 can penetrate beneath the skin of the biological sample 103. The depth to which the electrical stimulating signals 111 penetrate can be controlled by controlling one or more parameters of the electrical stimulating signals 111.

The optical device 109 is configured to detect the optically detectable changes within the biological sample 103. In the example of Fig. 3 the optical device 109 comprises an OCT device. Other types of optical device could be used in other examples of the disclosure.

The optical device 109 can comprise a light source that is configured to generate an emitted beam of light. Where the optical device 109 comprises an OCT device the emitted beam of light can comprise one or more low coherence beams of light. The one or more low coherence beams of light can be any suitable wavelength.

The beam of light that is emitted by the light source can comprise any suitable wavelength. Where the optical device 109 comprises an OCT device, the light source can use near infrared light to scan part of the biological sample 103. The OCT device can use light of a wavelength between 850nm and 1050nm. Other wavelengths of light could be used in other examples.

If an OCT device is used, the OCT device could be a speckle-modulated OCT which is sensitive to small morphology changes, or a polarization-sensitive OCT which can highlight birefringence of nerve tissue or other suitable part of the biological sample 103 or any other suitable type of OCT device.

The optical device 109 can also comprise one or more sensors that are configured to detect light that has been reflected or scattered from the biological sample 103. The light from the optical device 109 that is incident on the biological sample 103 can be reflected or scattered by internal structures within the biological sample 103 such as the sensory receptors 113. The amount of the light that is reflected or scattered can be dependent upon one or more physical characteristics of the sensory receptors 113 and/or other parts of the biological sample 103. The amount of the light that is reflected or scattered, and other parameters of the light that is reflected or scattered, can be dependent upon the properties of the internal structures within the biological sample 103. The properties can change in response to the application of the electrical stimulating signals 111. For example, the depolarization and the repolarization of the axon 117 of the sensory receptors 113 as a pain signal is passed through sensory receptor 113 can affect the light that is reflected or scattered by the biological sample 103.

In this example the optical device 109 is a small and lightweight device. The optical device 109 could comprise optical assemblies that have a diameter of the order of a few millimeters. This can enable the optical device 109 to be moved around and used to scan different parts of the biological sample 103. In some examples the optical device 109 can comprise the optical components, such as the light emitter and the light sensors, on a single chip or in a chip-sized arrangement.

In the example of Fig. 3 the optical device 109 comprises a probe shaped device that can be positioned adjacent to the forearm of the subject. The probe shaped device could be handled by a medical practitioner or any other suitable user.

Other form factors for the optical device 109 could be used in other examples of the disclosure. For instance, in some examples the optical assembly could comprise optimally arranged optical conduits or components that could be positioned around, at least part of, the surface of a subject's limb or any other suitable part of their body. The positioning of the light source and the optical conduits or components can be arranged to enable any suitable parts of a biological sample 103 to be monitored. The optical device 109 can be positioned relative to the stimulator 107 so that the optical device 109 can scan the part of the biological sample 103 that has been stimulated by the electrical stimulating signals 111.

The measurements obtained by the optical device 109 can be provided to an apparatus 101. The apparatus 101 can then use the measurements from the optical device and the respective values for the one or more parametersof the electrical stimulating signals 111 that were used to generate a pain profile for the biological sample 103, for example respective power levels of the electrical stimulating signals.

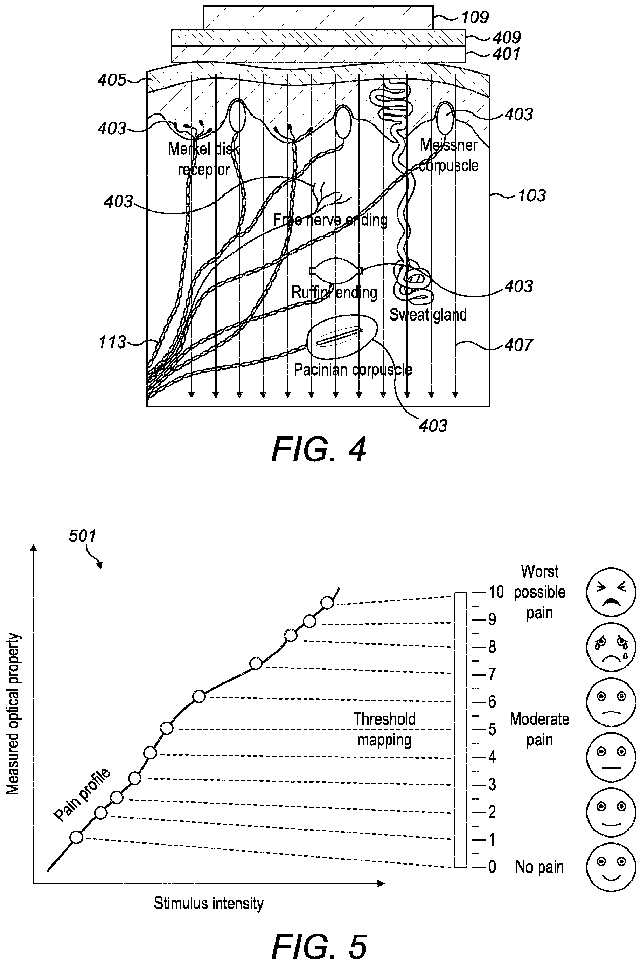

Fig. 4 shows a biological sample 103 being scanned by an optical device 109.

The biological sample 103 can comprise any suitable part of a subject. For example, the biological sample 103 can comprise a subject's forearm or any other suitable part of a subject.

The biological sample 103 comprises a plurality of internal structures 403. The internal structures are positioned underneath the skin 405 of the subject. In this example the internal structures comprise Merkel disk receptors, Meissner corpuscles, Free nerve endings, Ruffini endings, Pacinian Corpuscles, and any other suitable internal structure. The number and arrangement of these internal structures will vary between different biological samples 103.

The internal structures 403 can have inherent optical properties. These optical properties can comprise refractive index, birefringence, bioluminescence or any other suitable parameter. These inherent optical properties can be temporarily modified by the application of an electrical stimulating signal and/or by the subject experiencing pain.

In the example of Fig. 4 the optical device 109 comprises a light source 401 and an optical detector 409. The optical device 109 can comprise additional components in other examples. The optical device 109 could comprise a plurality of light sources 401 and a plurality of optical detectors 409.

The light source 401 of the optical device 109 is configured to scan the biological sample 103. The light source 401 provides light 407 at any suitable wavelength. The light 407 can be provided at a wavelength that allows it to penetrate the skin 405 of the biological sample 103. The light 407 can be provided at a wavelength that reflects or scatters off one or more of the internal structures 403 to enable images or other information of the internal structures 403 to be obtained.

The optical detector 409 is configured to detect light reflected or scattered from the biological sample 103. This light can provide quantitative information about the optical properties of the internal structures 403 within the biological sample 103.

The light can also provide an indication of any changes within the internal structure 403 of the biological sample 103. These changes can be analysed to generate a pain profile of the biological sample 103. The information can comprise information indicative of both the location of the changes and the magnitude of the changes. Once the pain profile has been generated the changes can be monitored to assess pain levels being experienced within the biological sample 103.

Fig. 5 shows an example pain profile 501 that can be generated using examples of the disclosure.

The pain profile comprises a plot associating the power of stimulus on the x-axis to a measured optical response on the y-axis. The stimulus could be the electrical stimulating signals 111 used to generate the pain profile 501 or any other suitable type of extremal stimulus. The external stimulus could be something that causes pain for the subject.

The measured optical property could comprise one or more of refractive index, birefringence, fluorescence of any one or more of the internal structures 403 within the biological sample 103.

The parts of the pain profile 501 that correspond to a low stimulus power can be generated using the electrical stimulating signals 111. These can relate to stimuli which cause the subject no pain or very little pain. The rest of the pain profile can be generated using extrapolation or any suitable process.

The pain profile 501 in the example of Fig. 5 comprises a pain scale where the pain scale comprises a plurality of different pain bands. The pain bands within the pain scale are assigned a numerical value from 0 to 10. The numerical band 0 represents the subject experiencing no pain and the numerical band 10 represents the subject experiencing the worst possible pain. The bands between 0 to 10 represent increasing levels of pain. Any other suitable band intervals can be used in combination with the proposed pain profile, to augment pain reporting for a subject using both quantitative and qualitative measurements.

The pain profile 501 provides an indication of the expected measurement of the optical property at each of these different bands. The pain profile 501 can be used to assess the pain being experienced by a subject by scanning the biological sample 103 using the optical device 109 to measure the optical properties of the biological sample 103. The values of the measured optical properties can then be identified on the pain profile 501 and associated with one of the bands between 0 to 10.

This pain profile 501 therefore has a wide range of uses. For example, it can enable a medical practitioner to determine how much pain a subject is experiencing and/or can enable the location of the pain to be determined. This can be more reliable than relying on self-reporting by a subject which can be subjective and inaccurate. Also, self-reporting might not be available for some subjects such as animals, young children or non-verbal subjects.

For instance, the pain profile 501 could be used to monitor a subject suffering from chronic or long-term pain. To enable such monitoring a subject could wear an optical device 109 to monitor the optical properties of one or more parts of the subject, such as their forearm or any other suitable location. These optical properties can then be associated with a pain band within the pain profile 501 to identify the levels of pain experienced by the subject. This can enable the levels of pain experienced by the subject to be monitored over extended periods of time. This can be more accurate and reliable compared to relying on the subject's recollection of the experience of the pain.

Examples of the disclosure can therefore be used by medical practitioners, or other suitable users to more accurately identify the level of pain being experienced by a subject. This can be used to assist with diagnosing medical issues. In some examples it could be used to distinguish phantom pain or psychological pain from other types of pain. This could also be used to identify the location of the source of pain and could be used to help resolve issues if there are multiple locations of the source of pain.

In some examples the pain profile 501 and the monitoring of the optical properties could be used to monitor a subject recovering from an injury and/or undergoing rehabilitation. Such patients might have impaired ability to feel the pain and monitoring the optical properties can help to prevent further damage or injury to the subject.

Examples of the disclosure also enable an individual pain profile 501 to be generated for a subject. This could be unique to the subject. This could then be used to take into account that different subjects respond differently to pain. For example, a subject who is more sensitive to pain could have different settings for the bands in the pain scale in the pain profile 501 compared to the bands in a pain profile for a subject with a lower sensitivity to pain,

Fig. 6 schematically illustrates an apparatus 101 that can be used to implement examples of the disclosure. In this example the apparatus 101 comprises a controller 601. The controller 601 illustrated in Fig. 6 can be a chip or a chip-set. In some examples the controller 601 can be provided within an electronic system 105 that is configured to provide stimulating electrical signals to a biological sample 103. In some examples the controller 601 can be provided externally to the electronic system 105 but can be configured to provide electronic signals to enable control of the electronic system 105 and/or can receive signals indicative of data obtained by the electronic system 105.

In the example of Fig. 6 the implementation of the controller 601 can be as controller circuitry. In some examples the controller 601 can be implemented in hardware alone, have certain aspects in software including firmware alone or can be a combination of hardware and software (including firmware).

As illustrated in Fig. 6 the controller 601 can be implemented using instructions that enable hardware functionality, for example, by using executable instructions of a computer program 607 in a general-purpose or special-purpose processor 603 that can be stored on a computer readable storage medium (disk, memory etc.) to be executed by such a processor 603.

The processor 603 is configured to read from and write to the memory 605. The processor 603 can also comprise an output interface via which data and/or commands are output by the processor 603 and an input interface via which data and/or commands are input to the processor 603.

The memory 605 is configured to store a computer program 607 comprising computer program instructions (computer program code 609) that controls the operation of the controller 601 when loaded into the processor 603. The computer program instructions, of the computer program 607, provide the logic and routines that enables the controller 601 to perform the methods illustrated in Fig. 2. The processor 603 by reading the memory 605 is able to load and execute the computer program 607.

The apparatus 101 therefore comprises: at least one processor 603; and at least one memory 605 including computer program code 609, the at least one memory 605 and the computer program code 609 configured to, with the at least one processor 603, cause the apparatus 101 at least to perform:
enabling stimulation of a biological sample using a plurality of electrical stimulating signals comprising different values for one or more parameters wherein respective electrical stimulating signals are configured to cause a respective change in the biological sample wherein the respective change is optically detectable;
enabling measuring of the optically detectable changes caused by the electrical stimulating signals by using one or more optical devices to detect the optically detectable changes; and
using the measurements obtained by the one or more optical devices and the corresponding values for the one or more parameters of the electrical stimulating signals to generate a pain profile for the biological sample based on the optically detectable changes caused by the stimulation.

As illustrated in Fig. 6 the computer program 607 can arrive at the controller 601 via any suitable delivery mechanism 611. The delivery mechanism 611 can be, for example, a machine readable medium, a computer-readable medium, a non-transitory computer-readable storage medium, a computer program product, a memory device, a record medium such as a Compact Disc Read-Only Memory (CD-ROM) or a Digital Versatile Disc (DVD) or a solid-state memory, an article of manufacture that comprises or tangibly embodies the computer program 607. The delivery mechanism can be a signal configured to reliably transfer the computer program 607. The controller 601 can propagate or transmit the computer program 607 as a computer data signal. In some examples the computer program 607 can be transmitted to the controller 601 using a wireless protocol such as Bluetooth, Bluetooth Low Energy, Bluetooth Smart, 6LoWPan (IPᵥ6 over low power personal area networks) ZigBee, ANT+, near field communication (NFC), Radio frequency identification, wireless local area network (wireless LAN) or any other suitable protocol.

The computer program 607 comprises computer program instructions for causing an apparatus 101 to perform at least the following:
enabling stimulation of a biological sample using a plurality of electrical stimulating signals comprising different values for one or more parameters wherein respective electrical stimulating signals are configured to cause a respective change in the biological sample wherein the respective change is optically detectable;
enabling measuring of the optically detectable changes caused by the electrical stimulating signals by using one or more optical devices to detect the optically detectable changes; and
using the measurements obtained by the one or more optical devices and the corresponding values for the one or more parameters of the electrical stimulating signals to generate a pain profile for the biological sample based on the optically detectable changes caused by the stimulation.

The computer program instructions can be comprised in a computer program 607, a non-transitory computer readable medium, a computer program product, a machine readable medium. In some but not necessarily all examples, the computer program instructions can be distributed over more than one computer program 607.

Although the memory 605 is illustrated as a single component/circuitry it can be implemented as one or more separate components/circuitry some or all of which can be integrated/removable and/or can provide permanent/semi-permanent/ dynamic/cached storage.

Although the processor 603 is illustrated as a single component/circuitry it can be implemented as one or more separate components/circuitry some or all of which can be integrated/removable. The processor 603 can be a single core or multi-core processor.

References to "computer-readable storage medium", "computer program product", "tangibly embodied computer program" etc. or a "controller", "computer", "processor" etc. should be understood to encompass not only computers having different architectures such as single /multi- processor architectures and sequential (Von Neumann)/parallel architectures but also specialized circuits such as field-programmable gate arrays (FPGA), application specific circuits (ASIC), signal processing devices and other processing circuitry. References to computer program, instructions, code etc. should be understood to encompass software for a programmable processor or firmware such as, for example, the programmable content of a hardware device whether instructions for a processor, or configuration settings for a fixed-function device, gate array or programmable logic device etc.

As used in this application, the term "circuitry" can refer to one or more or all of the following:
(a) hardware-only circuitry implementations (such as implementations in only analog and/or digital circuitry) and
(b) combinations of hardware circuits and software, such as (as applicable):
   (i) a combination of analog and/or digital hardware circuit(s) with software/firmware and
   (ii) any portions of hardware processor(s) with software (including digital signal processor(s)), software, and memory(ies) that work together to cause an apparatus, such as a mobile phone or server, to perform various functions and
(c) hardware circuit(s) and or processor(s), such as a microprocessor(s) or a portion of a microprocessor(s), that requires software (e.g. firmware) for operation, but the software cannot be present when it is not needed for operation.

This definition of circuitry applies to all uses of this term in this application, including in any claims. As a further example, as used in this application, the term circuitry also covers an implementation of merely a hardware circuit or processor and its (or their) accompanying software and/or firmware. The term circuitry also covers, for example and if applicable to the particular claim element, a baseband integrated circuit for a mobile device or a similar integrated circuit in a server, a cellular network device, or other computing or network device.

The blocks illustrated in Fig. 2 can represent steps in a method and/or sections of code in the computer program 607. The illustration of a particular order to the blocks does not necessarily imply that there is a required or preferred order for the blocks and the order and arrangement of the blocks can be varied. Furthermore, it can be possible for some blocks to be omitted.

The term 'comprise' is used in this document with an inclusive not an exclusive meaning. That is any reference to X comprising Y indicates that X may comprise only one Y or may comprise more than one Y. If it is intended to use 'comprise' with an exclusive meaning then it will be made clear in the context by referring to "comprising only one..." or by using "consisting".

In this description, reference has been made to various examples. The description of features or functions in relation to an example indicates that those features or functions are present in that example. The use of the term 'example' or 'for example' or 'can' or 'may' in the text denotes, whether explicitly stated or not, that such features or functions are present in at least the described example, whether described as an example or not, and that they can be, but are not necessarily, present in some of or all other examples. Thus 'example', 'for example', 'can' or 'may' refers to a particular instance in a class of examples. A property of the instance can be a property of only that instance or a property of the class or a property of a sub-class of the class that includes some but not all of the instances in the class. It is therefore implicitly disclosed that a feature described with reference to one example but not with reference to another example, can where possible be used in that other example as part of a working combination but does not necessarily have to be used in that other example.

Although examples have been described in the preceding paragraphs with reference to various examples, it should be appreciated that modifications to the examples given can be made without departing from the scope of the claims.

Features described in the preceding description may be used in combinations other than the combinations explicitly described above.

Although functions have been described with reference to certain features, those functions may be performable by other features whether described or not.

Although features have been described with reference to certain examples, those features may also be present in other examples whether described or not.

The term 'a' or 'the' is used in this document with an inclusive not an exclusive meaning. That is any reference to X comprising a/the Y indicates that X may comprise only one Y or may comprise more than one Y unless the context clearly indicates the contrary. If it is intended to use 'a' or 'the' with an exclusive meaning then it will be made clear in the context. In some circumstances the use of 'at least one' or 'one or more' may be used to emphasis an inclusive meaning but the absence of these terms should not be taken to infer any exclusive meaning.

The presence of a feature (or combination of features) in a claim is a reference to that feature or (combination of features) itself and also to features that achieve substantially the same technical effect (equivalent features). The equivalent features include, for example, features that are variants and achieve substantially the same result in substantially the same way. The equivalent features include, for example, features that perform substantially the same function, in substantially the same way to achieve substantially the same result.

In this description, reference has been made to various examples using adjectives or adjectival phrases to describe characteristics of the examples. Such a description of a characteristic in relation to an example indicates that the characteristic is present in some examples exactly as described and is present in other examples substantially as described.

Whilst endeavoring in the foregoing specification to draw attention to those features believed to be of importance it should be understood that the Applicant may seek protection via the claims in respect of any patentable feature or combination of features hereinbefore referred to and/or shown in the drawings whether or not emphasis has been placed thereon.

## Claims

1. An apparatus comprising means for:
enabling stimulation of a biological sample using a plurality of electrical stimulating signals comprising different values for one or more parameters wherein respective electrical stimulating signals are configured to cause a respective change in the biological sample wherein the respective change is optically detectable;
enabling measuring of the optically detectable changes caused by the electrical stimulating signals by using one or more optical devices to detect the optically detectable changes; and
using the measurements obtained by the one or more optical devices and the corresponding values for the one or more parameters of the electrical stimulating signals to generate a pain profile for the biological sample based on the optically detectable changes caused by the stimulation.

2. An apparatus as claimed in claim 1 wherein the one or more optical devices used to detect the optically detectable changes comprise one or more optical coherence tomography devices.

3. An apparatus as claimed in any preceding claim wherein the measurements obtained by the one or more optical devices are used to generate the pain profile for a region of the pain profile below a threshold level of the electrical stimulating signal and extrapolation of data is used to generate the pain profile for a region above the threshold level of the electrical stimulating signal.

4. An apparatus as claimed in any preceding claim wherein the one or more parameters of the electrical stimulating signals comprises power.

5. An apparatus as claimed in any preceding claim wherein the generated pain profile is specific to the biological sample.

6. An apparatus as claimed in any preceding claim wherein the stimulation of the biological sample is spatially modulated so as to target one or more regions of the biological sample.

7. An apparatus as claimed in any preceding claim wherein the respective optically detectable changes caused by the electrical stimulating signals comprise one or more cellular or biomolecular level events.

8. An apparatus as claimed in claim 7 wherein the cellular or biomolecular level events comprise one or more of; cellular depolarization, transmembrane voltage changes, changes in nervous tissue, electrolytic chemical imbalance.

9. An apparatus as claimed in any preceding claim wherein the optically detectable changes comprise a change in one or more optical properties of the biological sample.

10. An apparatus as claimed in any preceding claim wherein the measurements obtained by the one or more optical devices and the corresponding values for the one or more parameters of the electrical stimulating signals are used in combination with one or more qualitative pain indicators to generate the pain profile for the biological sample

11. An apparatus as claimed in any preceding claim wherein the means are for controlling the electrical stimulating signals to vary one or more of frequency, duration, cycle, pattern, shape of the electrical stimulating signals.

12. An apparatus as claimed in any preceding claim comprising means for obtaining observation measurements from an optical device and using the obtained observation measurements and the generated pain profile to determine a pain level of the biological sample.

13. An electronic device comprising an apparatus as claimed in any preceding claim.

14. A method comprising:
enabling stimulation of a biological sample using a plurality of electrical stimulating signals comprising different values for one or more parameters wherein respective electrical stimulating signals are configured to cause a respective change in the biological sample wherein the respective change is optically detectable;
enabling measuring of the optically detectable changes caused by the stimulating signals by using one or more optical devices to detect the optically detectable changes; and
using the measurements obtained by the one or more optical devices and the corresponding values for the one or more parameters of the electrical stimulating signals to generate a pain profile for the biological sample based on the optically detectable changes caused by the stimulation.

15. A computer program comprising computer program instructions that, when executed by processing circuitry, cause:
enabling stimulation of a biological sample using a plurality of electrical stimulating signals comprising different values for one or more parameters wherein respective electrical stimulating signals are configured to cause a respective change in the biological sample wherein the respective change is optically detectable;
enabling measuring of the optically detectable changes caused by the stimulating signals by using one or more optical devices to detect the optically detectable changes; and
using the measurements obtained by the one or more optical devices and the corresponding values for the one or more parameters of the electrical stimulating signals to generate a pain profile for the biological sample based on the optically detectable changes caused by the stimulation.
